# EUROPEAN PATENT APPLICATION

(11) **EP 4 772 204 A1**
(43) Date of publication of application: **08.07.2026**
(21) Application number: 25150477.5
(22) Date of filing: 07.01.2025
(51) Int. Cl.: A61M 1/06

(54) **IN-BRA SYSTEM FOR EXPRESSING BREAST MILK WITH IMPROVED NIPPLE VISIBILITY**

(71) Applicant: Koninklijke Philips N.V., 5656 AE Eindhoven (NL)
(72) Inventor: LUI, Kwan Fai, Eindhoven (NL); CARTEI, Mirko, Eindhoven (NL); LI, Song Lin, Eindhoven (NL)
(74) Representative: Philips Intellectual Property & Standards

(57) **Abstract**

A breast pump for expressing milk from a nipple of a breast, the breast pump comprising a pump system to provide a pressure profile to be applied to at least a part of the breast and wherein the breast pump is configured to be received in a user's bra during use,
characterized in that,
the breast pump is configured such that, during use, no part of the breast pump blocks a user's unobstructed view of the nipple.

## Description

### FIELD OF THE INVENTION

The invention relates to a breast pump for expressing milk from a nipple of a breast, particularly a human female breast. The invention also relates to a milk container for such a breast pump and a pump unit for such a breast pump.

### BACKGROUND OF THE INVENTION

An example of such a breast pump, such a milk container, and such a pump unit is provided by the Momcozy All-in-one M5 wearable breast pump. This breast pump comprises:
- a breast shield configured to interface with a breast;
- a milk collector configured to receive expressed milk and whose internal volume, during use, is partially occupied by the breast shield, and
- a main unit comprising the pump motor.

The breast pump is designed such that, during use, the main unit rests on top of the milk collector.

As another example, the Momcozy S 12 breast pump shows a design similar to that of the Momcozy M5 breast pump.

US20210196874A1 shows a slightly different, but still similar designed breast pump. This breast pump comprises:
- a breast shield configured to interface with a breast;
- a milk collector configured to receive expressed milk, and
- a main unit comprising the pump motor.

With the breast pump of US20210196874A1 it is a volume defined by the main unit (not the milk container) that, during use, is partially occupied by the breast shield. However, like the Momcozy breast pumps, the breast pump of US20210196874A1 is designed such that, during use, the main unit rests on top of the milk collector.

All the above designs result in breast pumps that do not allow a user an unobstructed view of a nipple of the breast during use, particularly at the start and during the early phases of a milk expression session. During these periods at least one of (i) proper positioning of the breast pump on the breast and (ii) being able to see whether a milk letdown reflex occurs may be relevant to the user.

### SUMMARY OF THE INVENTION

It is an object of the invention to provide a breast pump for expressing milk from a nipple of a breast, the breast pump comprising a pump system to provide a pressure profile to be applied to at least a part of the breast and the breast pump being configured to be received in a user's bra during use, that provides a user with a view of the nipple during use.

According to the invention, this object is realized in that the breast pump is configured such that, during use, no part of the breast pump blocks a user's unobstructed view of the nipple. This means that:
(i) parts of the breast pump that would otherwise interfere with the user's view of the nipple are positioned (or repositioned (compared to known devices)) such that they do not interfere with the user's view of the nipple;
(ii) parts of the breast pump that would otherwise interfere with the user's view of the nipple have been made to be visually transparent; or
(iii) both (i) and (ii).

A part being visually transparent means that, if the part is put on a line of sight between the user to the nipple, the part allows the user to visually observe the nipple.

Repositioning parts of the breast pump that would otherwise interfere with the user's view of the nipple comprises that, during use, these parts are only positioned in at least one of the following positions:
(a) below a volume that, during use, is occupied by at least a part of the breast;
(b) laterally from a volume occupied by the nipple as seen from the user's perspective;
(c) distally from a volume occupied by the nipple as seen from the user's perspective; and
(d) proximally from a volume occupied by the nipple as seen from the user's perspective.

In each of (a)-(d) the parts are positioned to not be in front of the nipple as seen from the user's perspective during use. In embodiments each of these volumes is defined by physical boundaries comprised in the breast pump.

Reasons that contribute to the arrangements used in the prior art may that putting a milk collector at a lower position than other parts during use, allows the use of gravity to let milk flow from a nipple towards the milk collector. Moreover, putting a main unit comprising a pump motor at a higher position than other parts during use, allows a user easy access to a user interface comprised in the main unit. Having the main unit comprise the user interface is convenient because the main unit already comprises the pump motor and is likely to further comprise other parts of a pump system, for instance electronics and batteries. With these components in the main unit, it is easy to add a user interface.

According to an embodiment, the breast is configured such that, during use,
(1) the breast pump comprises a nipple chamber for receiving the nipple,
(2) the nipple chamber comprises a first visually transparent part, wherein the first visually transparent part is positioned to allow a user a view of a nipple received in the nipple chamber.

In embodiments a nipple chamber is defined by at least a part of a breast shield, a part comprising a volume to receive expressed milk or both the breast shield and the part together. Alternatively, defining the nipple chamber may also involve additional parts of the breast pump or other parts of the breast pump. However, regardless of the exact part or parts of the breast pump involved in defining the nipple chamber, the nipple chamber comprising a first visually transparent part allows a user a view of the nipple during use, while being able to apply a pressure profile to at least a part of the breast. The first visually transparent part may be the only object on the line of sight between a user and the nipple. The first visually transparent part is then part of an outer boundary of the breast pump. However, the first visually transparent part may be positioned in a recess in the breast pump to limit the volume of the nipple chamber while having sufficient volume in the rest of the breast pump to position other parts and/or to create an outer shape that allows the breast pump to be received in a user's bra during use.

According to an embodiment, the breast pump comprises an outer wall portion, not part of the nipple chamber and defining an outer boundary of the breast pump, and wherein the outer wall portion comprises a second visually transparent part, wherein the breast pump is configured such that, during use, the first visually transparent part and the second visually transparent part are aligned to allow the user the unobstructed view of the nipple.

According to an embodiment, the breast pump is configured such that, during use, the pump system is positioned to not interfere with the unobstructed view. In embodiments, the positioning comprises one or more of the options (a)-(d) listed above. In the prior art devices described above the main unit, i.e. the unit comprising the pump motor, blocks a user's view of the nipple from which milk is to be expressed. After all, in the Momcozy breast pumps, the main unit rests on the milk collector that, during use, also holds the breast shield and at least a part of the breast received by the breast shield. As the main unit is not transparent, it blocks the user's view of the nipple. in the breast pump of US20210196874A1, the main unit defines a volume that, during use, is occupied by a at least a part of a breast shield and at least a part of a breast from which milk is to be expressed, the at least part of the breast having been received in the breast shield. As the main unit is not transparent, it blocks the user's view of the nipple.

According to an embodiment, the breast pump comprises a milk chamber for receiving expressed milk and wherein the milk chamber is configured to at least partially extend below a volume for receiving the nipple during use. Having the milk chamber extend below the nipple allows the use of gravity to have milk flow from the nipple into the milk chamber.

However, the milk chamber need not be configured to at least partially extend below a volume for receiving the nipple during use. In embodiments, the milk chamber does not extend below the nipple chamber.

In embodiments the volume of the milk chamber, regardless of whether it extends lower than the nipple chamber, is not more than 100, 150, 180, 200, 220, 250, 275, and 300 ml. In embodiments the volume of the milk chamber lower than the nipple chamber comprises a volume of not more than 10, 20, 50, 100, 120, 150, 180, 200, 220, 250, or 280 ml. In embodiments the volume of the milk chamber lower than the nipple chamber comprises not more than 5%, 10%, 20%, 30%, 40%, 50%, 60%, 70% of the volume of the milk chamber. The term 'lower' comprises both `directly beneath' the nipple chamber and `at a height lower than the nipple chamber' (but optionally more lateral than the nipple tunnel).

According to an embodiment, the milk chamber comprises a protruding part or volume configured to extend below a volume for receiving the nipple during use and wherein, during use, at least a part of the pump system at least partially surrounds the protrusion. Having a protruding part of the milk chamber extend below the nipple allows the use of gravity to have milk flow from the nipple into the protruding part, while creating space for other parts of the breast pump to one or more sides of the protruding part. The shape of the milk chamber is adapted such that the volume of the part of the milk chamber lower than the nipple is reduced to make space available to other parts of the breast pump that would, during use, interfere with a user's view of her nipple if these other parts had been positioned on a line of sight between the user and the nipple.

According to an embodiment, the breast pump comprises a breast unit configured to receive the at least part of the breast and a pump unit comprising the pump system, wherein the pump unit is not comprised in the breast unit. This embodiment has the advantage that the pump system is spatially separated or separable from the section of the breast pump that is configured to receive at least a part of a breast from which milk is to be expressed, i.e. the breast unit. This simplifies configuring the breast unit such that it allows a user an unobstructed view of her nipple during use. For instance, the breast unit may be made to be visually transparent, either in whole or in part.

According to an embodiment, the breast unit and the pump unit are removably attachable to each other. The breast unit and the pump unit being removably attachable to each other helps in simplifying cleaning of the breast unit.

According to an embodiment, the breast unit comprises the milk chamber. This embodiment removes the need for a separate milk chamber and, consequently, allows to reduce the number of parts that need to be manufactured and assembled prior to use.

According to an embodiment, the milk chamber is defined by a breast shield and a milk container, wherein the breast shield and the milk container are removably attachable to each other. This embodiment has the advantage that the breast unit comprises a limited number of components, making manufacturing and assembly prior to use easier. The milk container provides a holder for at least a part of the breast shield and the breast shield acts as a lid on the milk container, thereby defining the milk chamber.

According to an embodiment, the breast pump comprises a membrane configured to communicate the pressure profile to the at least part of the breast during use and wherein, during use, the membrane is positioned to not interfere with the unobstructed view. The breast pump of US20210196874A1 comprises a membrane that, during use, is positioned above the part of the breast shield configured to receive at least a part of a breast. The current embodiment specifies positioning of the membrane such that it does not interfere with an unobstructed view of the nipple. In embodiments, the positioning comprises one or more of the options (a)-(d) listed above.

According to an embodiment, the breast pump comprises a user interface configured to allow the user to:
(i) control an operation of the breast pump;
(ii) receive information from the breast pump, or

control an operation of the breast pump and receive information from the breast pump,
wherein, during use, the user interface is positioned to not interfere with the unobstructed view.

The breast pump of US20210196874A1 comprises a user interface that, during use, is positioned above the part of the breast shield configured to receive at least a part of a breast. The current embodiment specifies positioning of the membrane such that it does not interfere with an unobstructed view of the nipple. In embodiments, the positioning comprises one or more of the options (a)-(d) listed above.

According to an embodiment, the pump unit comprises the user interface. This embodiment has the advantage of limiting the number of parts to be assembled. In an embodiment, the breast pump comprises two main elements: first, a breast unit configured to receive at least a part of a breast and comprising a milk chamber and, second, a pump unit comprising the user interface.

The invention also relates to a milk container as defined above.

The invention also relates to a pump unit as defined above.

### BRIEF DESCRIPTION OF THE DRAWINGS

These and other aspects of the invention will be further elucidated and described with reference to the drawing, in which:
Fig. 1 schematically shows the Momcozy M5 breast pump (figure taken from manual, numbering amended).
Fig. 2 schematically shows the Momcozy S12 breast pump (figure taken from manual, numbering amended).
Fig. 3 schematically shows the breast pump from US20210196874A1.
Figs. 4A-4B schematically show lateral cross-sections of breast pumps according to the invention and comprising first visually transparent parts.
Fig. 5 schematically shows a lateral cross-section of a breast pump according to the invention and comprising a first visually transparent part and a second visually transparent part.
Fig. 6 schematically shows a user's view of the breast pumps of figures 4 and 5 during use.
Fig. 7 schematically shows a milk chamber according to the invention that is configured to at least partially extend lower than a volume for receiving the nipple during use.
Fig. 8 schematically shows a breast pump according to the invention comprising a breast unit and a pump unit.
Fig. 9A-9G schematically shows different relationships between a breast unit and a pump unit in a breast pump according to the invention.
Figs. 10A1-10B2 schematically show different relationships between breast units and pump units in breast pumps according to the invention, wherein the breast pumps comprise membranes that are positioned to not interfere with a users' view of nipples during use.

### DETAILED DESCRIPTION OF EXAMPLES

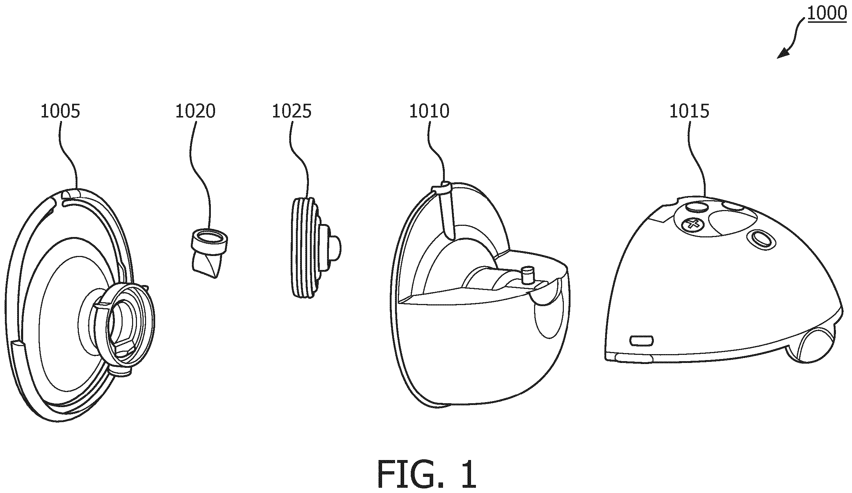

Fig. 1 schematically shows the Momcozy M5 breast pump (figure taken from manual, numbering amended). The breast pump (1000) comprises a breast shield (1005), a milk collector (1010), and a main unit (1015). During use the main unit (1015) essentially sits on top of the milk collector (1010), thereby blocking a user's view of a nipple placed inside the breast shield (1005), with the breast shield (1005) having been received in milk collector (1010). The breast pump further comprises a valve (1020) and a membrane (1025).

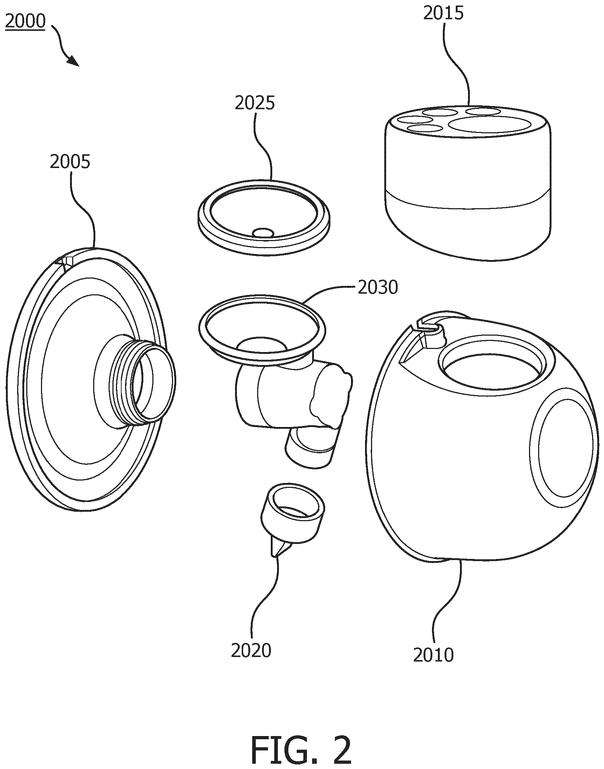

Fig. 2 schematically shows the Momcozy S12 breast pump (figure taken from manual, numbering amended). The breast pump (2000) comprises a breast shield (2005), a milk collector (2010), and a main unit (2015). During use the main unit (2015) sits on top of the milk collector (2010), thereby blocking a user's view of a nipple placed inside the breast shield (2005), with the breast shield (2005) having been received in milk collector (2005). The breast pump further comprises a valve (2020), a membrane (2025), and a linker (2030) for holding the valve (2020) and the membrane (2025).

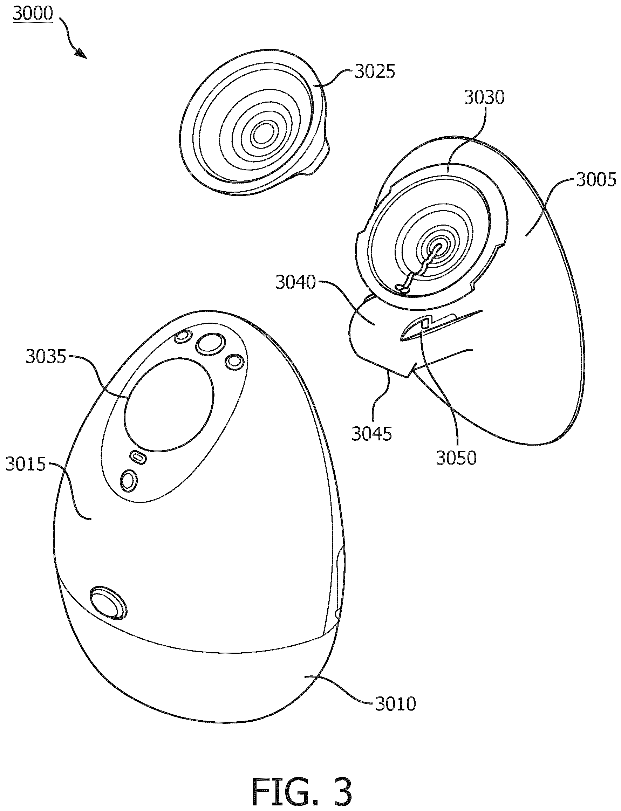

Fig. 3 schematically shows the breast pump from US20210196874A1. The breast pump (3000) comprises a breast shield (3005), a milk collector (3010), and a main unit (3015). During use the main unit (3015) sits on top of the milk collector (3010), thereby blocking a user's view of a nipple placed inside the breast shield (3005), with the breast shield (3005) having been received in main unit (3015). The breast pump further comprises a membrane (3025), a membrane holder (3030), a user interface (3035), a nipple chamber (3040) for receiving a nipple, a first opening (3045) for depositing milk into the milk collector (3010), and a pair of channels (3050) for holding in place the breast shield (3005).

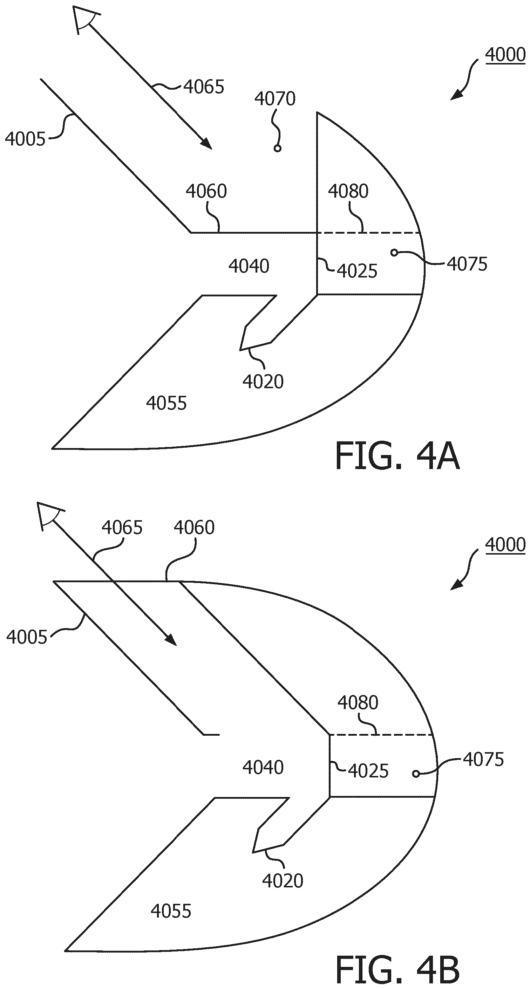

Figs. 4A-4B schematically show lateral cross-sections of breast pumps according to the invention and comprising first visually transparent parts. The breast pumps (4000) comprise breast shields (4005), valves (4020), membranes (4025), nipple chambers (4040), and milk chambers (4055). The nipple chambers (4040) comprise first visually transparent parts (4060). In this embodiment, the first visually transparent parts (4060) are the only elements in a users' views of nipples inside the nipple chambers (4040). These views are indicated by the lines of sight (4065).

The nipple chamber (4040) in fig. 4A is smaller than the nipple chamber (4040) shown in fig. 4B. Applying a pressure profile to a smaller nipple chamber is easier than applying the same pressure profile to a larger nipple chamber. In the latter case, more air has to be pumped. In fig. 4A the result of keeping nipple chamber (4040) relatively small is that a recess (4070) is formed.

The nipple chamber (4040) in fig. 4B is larger than the nipple chamber (4040) shown in fig. 4A. In fig. 4B the result of keeping nipple chamber (4040) relatively large is that no recess (4070) is needed and a continuous or, optionally, even a smooth outer shape of the breast pump (4000) can be achieved.

In all embodiments of the invention, the sizes inside the breast pumps (4000) of pump chambers (4075) can be defined as needed using wall portions (4080).

In all embodiments of the invention, a user is presented with an unobstructed view of a nipple in a nipple chamber. This means that parts of a breast pump that might interfere with this view are positioned such that they do not interfere with the view. For figs. 4A and 4B this means that such parts can be positioned, for instance, in at least one of positions (a)-(d) listed above. For instance, such parts can be positioned inside the breast pumps (4000) but below the nipple chambers (4040), laterally (including above, at, or below the level of nipple chambers (4040)), distally relative to nipple chambers (4040) as seen from a user's perspective during use, or proximally relative to nipple chambers (4040) as seen from a user's perspective during use. Such parts can also be positioned inside the breast pumps (4000) in any combination of the positions just mentioned, for instance, both below the level of nipple chambers (4040) and distally relative to nipple chambers (4040) as seen from a user's perspective during use.

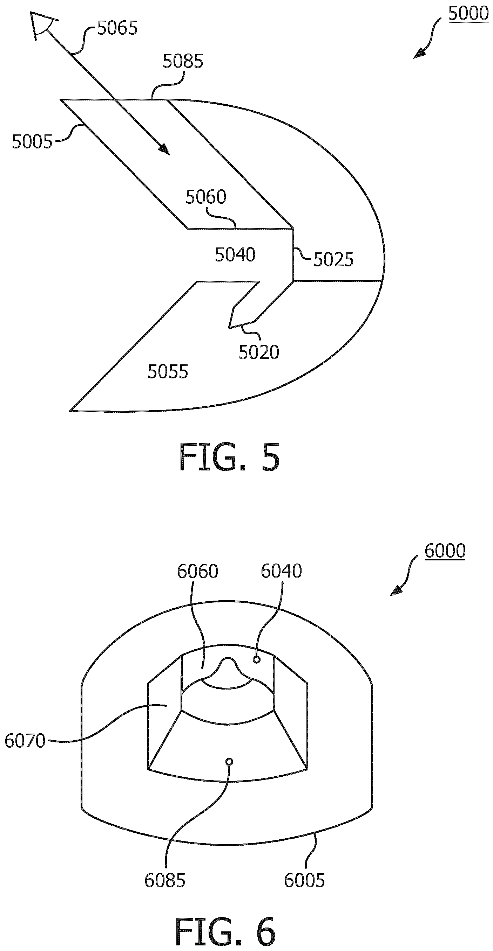

Fig. 5 schematically shows a lateral cross-section of a breast pump according to the invention and comprising a first visually transparent part and a second visually transparent part. The breast pump (5000) comprises a breast shield (5005), a valve (5020), a membrane (5025), a nipple chamber (5040), and a milk chamber (5055). The nipple chamber (5040) comprises a first visually transparent part (5060). The breast pump (5000) further comprises a second visually transparent part (5085). During use of the breast pump (5000), the first visually transparent part (5060) and the second visually transparent part (5085) are aligned such that they allow a user a view of a nipple inside the nipple chamber (5040) indicated by the line of sight (5065). In this embodiment the second visually transparent part (5085) is continuous with an outer contour of the breast pump (5000). The second visually transparent part (5085) may match the contour of a neighboring part of the breast pump (5000) resulting in a smooth continuous outer shape of the breast pump (5000) at the position of the second visually transparent part (5085) and the neighboring part.

As in all embodiments of the invention and as explained above, a user is presented with an unobstructed view of a nipple in a nipple chamber.

Fig. 6 schematically shows a user's view of the breast pumps of figures 4 and 5 during use. The breast pump (6000) comprises a breast shield (6005) and a nipple chamber (6040). The nipple chamber (6040) comprises a first visually transparent part (6060). The first visually transparent part (6060) is positioned in a recess (6070). The recess (6070) may comprise a second visually transparent part (6085), including at the top of the recess (6070), in which event the second visually transparent part (6085) is continuous with an outer shape of the breast pump (6000). The first visually transparent part (6060) and the second visually transparent part (6085) are aligned such that they allow a user a view of a nipple inside the nipple chamber (6040) during use (as shown in the figure).

As in all embodiments of the invention and as explained above, a user is presented with an unobstructed view of a nipple in a nipple chamber.

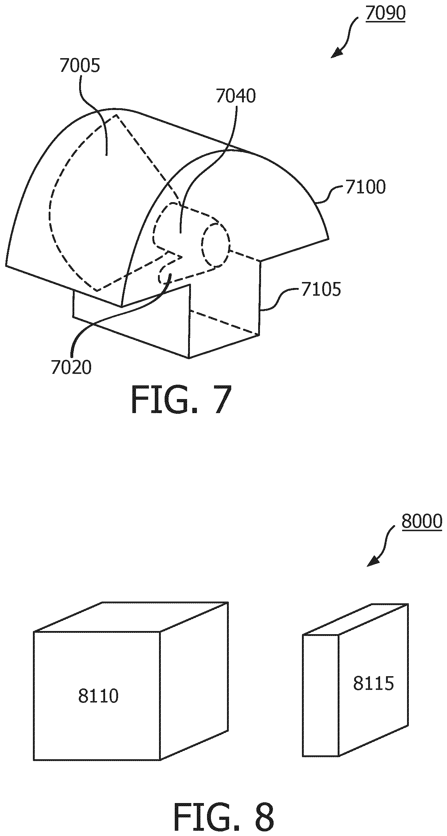

Fig. 7 schematically shows a milk chamber that is configured to at least partially extend lower than a volume for receiving the nipple during use. The apparatus (7090) comprises a breast shield (7005), a valve (7020), and a nipple chamber (7040). As the breast shield (7005) is configured to interface with a breast, it is to be positioned proximal relative to a user during use. The apparatus (7090) further comprises a section (7100) to be positioned distally relative to a user during use. The section (7100) comprises an extension (7105) that extends lower than the nipple chamber (7040). If the extension (7105) comprises a milk chamber or is itself comprised in a milk chamber for receiving expressed milk, the extension (7105) has the advantage that it enables the use of gravity to have expressed milk flow from a nipple towards the milk chamber. In fig. 7 the whole volume of the section (7100) not occupied by the breast shield (7005), valve (7020), nipple chamber (7040) is available to receive expressed milk. However, in an embodiment, only a part of the volume is accessible to expressed milk. The whole or a part of the top surface of the section (7100), i.e. the surface that in the embodiment of fig. 7 is curved, is transparent to allow a user a view of an inner volume of the section (7100). The top surface need not be curved. If only a part of the volume is accessible to expressed milk, the section (7100) may comprise one or more wall portions to limit the access. If a user is to be able to view the milk, at least a part of these one or more wall portions are visually transparent.

In fig. 7, the extension (7105) protrudes from the rest of section (7100) directly below the nipple chamber (7040). However, this is not essential. In other embodiments, the extension (7105) is positioned lower than the nipple chamber (7040), but also laterally relative to the nipple chamber (7040). In yet other embodiments, the extension (7105) is positioned lower than the nipple chamber (7040), but also laterally relative to the nipple chamber (7040), while still also extending directly below the nipple tunnel (7040). As long as the extension (7105) extends lower than the nipple chamber (7040) it enables the use of gravity to have expressed milk flow from a nipple towards the milk chamber and as long as the extension (7105) does not occupy the whole inner volume of a breast pump below a nipple chamber, it leaves room for other parts of a breast pump, for instance parts of a pump system configured to generate a pressure profile to be applied to at least a part of a breast. These other parts then do not have to be put in positions in which they would interfere with a user's view of a nipple during use. in fig. 7 such parts may be positioned to either lateral side of extension (7100) or to both lateral sides.

In fig. 7 the extension (7105) runs along the entire length of section (7100) in the proximal-distal direction. Of course this is not essential. If the extension (7100) does not run along the entire length, the space remaining along the length can again be used to position other parts of the breast pump. Thus, such other parts may be positioned at the level of the extension (7100) in space not occupied by the extension (7100). Thus, such other parts may at least partially surround the extension (7100).

The smaller the volume of the extension (7105), the sooner the liquid level of received expressed milk will rise as milk expression continues, which helps in making this level more easily visible to a user during use. After all, the higher the level, the closer the level is to higher parts of a breast pump (i.e. parts that are higher compared to other parts during use), the easier it is to allow the user a view of the milk level in such higher parts.

The breast shield (7005) and the section (7105) may be, but need not be, removably attachable to each other. If the breast shield (7005) and the section (7105) and are removably attachable, section (7105) can act as a holder for breast shield (7005) and the breast shield (7005) can act as a lid for a milk chamber comprised in the section (7105).

Fig. 8 schematically shows a breast pump comprising a breast unit and a pump unit. The breast pump (8000) comprises a breast unit (8110) configured to receive the at least part of the breast and a pump unit (8115) comprising the pump system, wherein the pump unit (8115) is not comprised in the breast unit (8110). Spatially separating hardware needed to receive at least a part of a breast and hardware needed, for instance, to create a pressure profile to be applied to the at least part of a breast simplifies configuring the breast unit (8110) to allow a user a view of a nipple during use.

The breast unit (8110) and the pump unit (8115) may be, but need not be, removably attachable to each other. Removable attachability simplifies cleaning of the breast unit (8110). The breast unit (8110) may take the form of the apparatus (7090) discussed in relation to fig. 7.

The pump unit (8115) shown in fig. 8 covers one side of the breast unit (8110) during use. This is not essential. The pump unit (8115) may cover less than one side of the breast unit (8110). In other embodiments the pump unit at least partially covers multiple sides of the breast unit (8115).

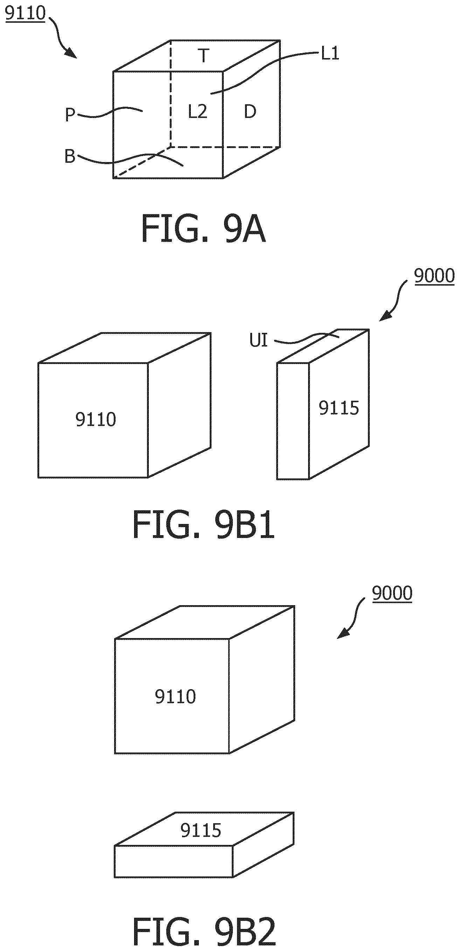

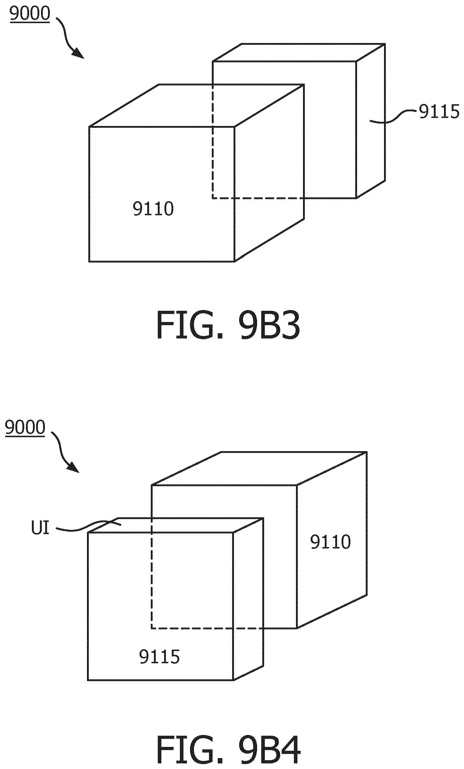

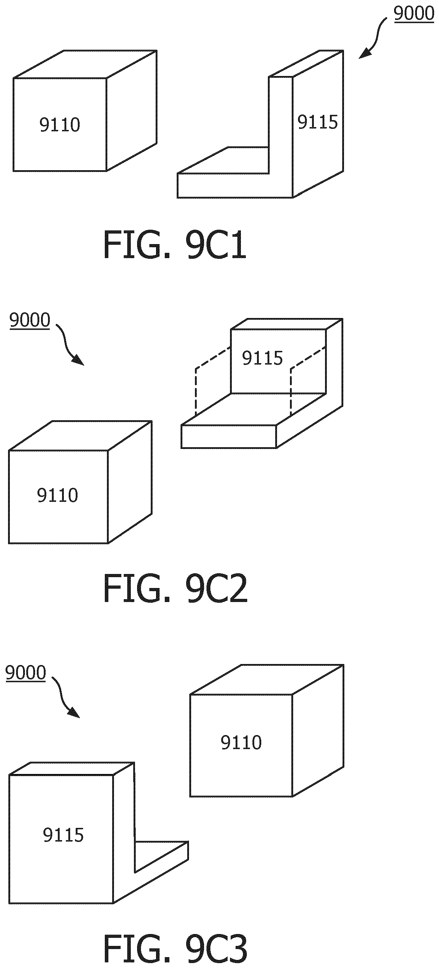

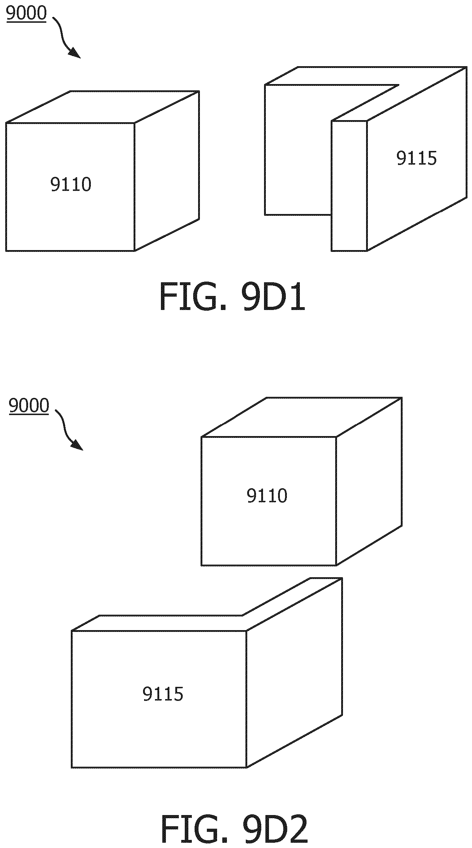

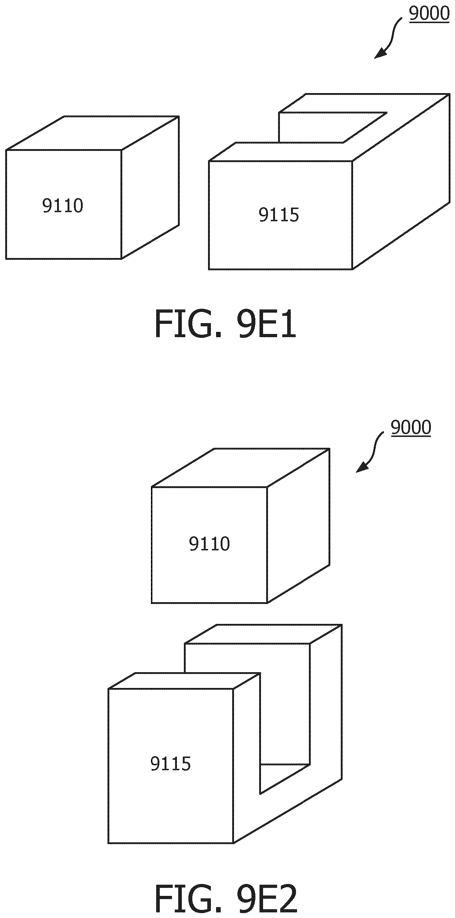

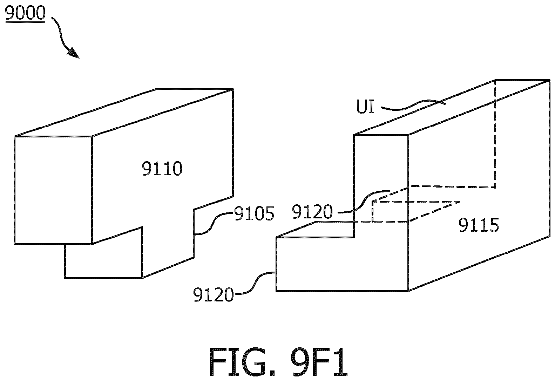

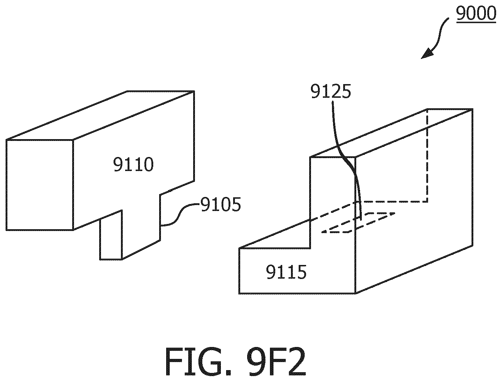

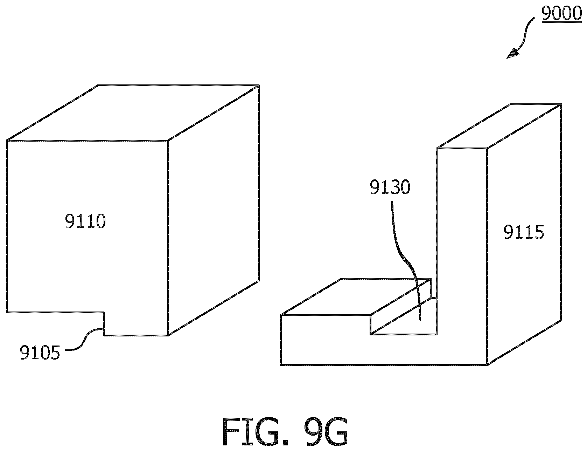

Figs. 9 schematically show different relationships between a breast unit and a pump unit in a breast pump according to the invention.

Fig. 9A first defines the different sides of a breast unit (9110). The breast unit (9110) has a proximal side that is facing a user during use. This proximal side is indicated by the letter P. The breast unit (9110) has a distal side opposite its proximal side. This distal side is indicated by the letter D. The breast unit (9110) has first and second lateral sides, one on each side of an axis in the proximal-distal direction. The first lateral side, facing away from the viewer, is indicated by L1. The second lateral side, facing the viewer, is indicated by L2. The breast unit (9110) has a top side. This top side is indicated by the letter T. The breast unit (9110) has a bottom side. This bottom side is indicated by the letter B.

Figs. 9B1-9B4 show a breast unit (9110) and a pump unit (9115), with the pump unit (9115) in the distal, bottom, first lateral, and second lateral positions respectively. The pump units (9115) shown in figs. 9B1-9B4 cover one side of the breast units (9110) during use. This is not essential. The pump units (9115) may cover at least a part of one side of the breast units (9110).

In all embodiments, the pump unit (9115) may even cover a part of the top side T of the breast unit (9110), as long as the pump unit (9115) does not interfere with a user's view of a nipple during use.

Figs. 9C1-9C3 show a breast unit (9110) and a pump unit (9115). The pump unit (9115) covers two sides of the breast unit (9110). Figs. 9C1-9C3 show the pump unit (9115) in the distal-bottom, first lateral-bottom, and second lateral-bottom positions respectively. The pump units (9115) shown in figs. 9C1-9C3 covers two sides of the breast units (9110) during use. This is not essential. The pump units (9115) may cover at least a part of each of the two sides of the breast units (9110). At least one of the sides of the pump units (9115) may comprise a guiding flange to assist in attaching the pump units (9115) and the breast units (9110) to each other. Such a guiding flange may, but need not, comprise an internal volume comprising parts of the breast pump (9000), such as parts of a pump system. By way of example dashed lines in fig. 9C2 schematically indicate two guiding flanges.

Figs. 9D1-9D2 show a breast unit (9110) and a pump unit (9115). The pump unit (9115) covers two sides of the breast unit (9110). Figs. 9D1-9D2 show the pump unit (9115) in the distal-first lateral and distal-second lateral positions respectively. The pump units (9115) shown in figs. 9D1-9D2 covers two sides of the breast units (9110) during use. This is not essential. The pump units (9115) may cover at least a part of each of the two sides of the breast units (9110). At least one of the sides, for instance two sides, of the pump units (9115) may comprise a guiding flange to assist in attaching the pump units (9115) and the breast units (9110) to each other. Such a guiding flange may, but need not, comprise an internal volume comprising parts of the breast pump (9000), such as parts of a pump system.

Figs. 9E1-9E2 show a breast unit (9110) and a pump unit (9115). The pump unit (9115) covers three sides of the breast unit (9110). Figs. 9E1-9E2 show the pump unit (9115) in the distal-first lateral-second lateral and bottom-first lateral-second lateral positions respectively. The pump units (9115) shown in figs. 9E1-9E2 cover three sides of the breast unit (9110) during use. This is not essential. The pump units (9115) may cover at least a part of each of the three sides of the breast unit (9110). At least one of the sides of the pump units (9115), for instance two or three sides, may comprise a guiding flange to assist in attaching the pump units (9115) and the breast units (9110) to each other. Such a guiding flange may, but need not, comprise an internal volume comprising parts of the breast pump (9000), such as parts of a pump system.

Figs. 9F1-9F2 show breast pumps (9000) comprising breast units (9110) and pump units (9115). The breast units (9110) comprise extensions (9105) like the extension (7105) discussed in relation to fig. 7. Reference is made here to fig. 7 and the corresponding discussion. In fig. 9F1, the pump unit (9115) comprises arms (9120) configured to laterally surround the extension (9105) during use. In fig. 9F1 the arms are not directly connected to each other, and both extend from the main body of pump unit (9115). However, this is not essential. In an embodiment the arms are directly connected, for instance by a flange that is configured to go underneath the extension (9105) at least during use. As discussed earlier, the pump unit (9115) may comprise a user interface, for instance on its top surface (indicated by `UI' in fig. 9F1) or on its distal surface or on both the top and distal surfaces.

In fig. 9F2 the extension (9105) runs along only a part of the length of the breast unit 99110) in the proximal-distal direction. The pump unit (9115) comprises a recess or a through hole (either indicated by 9125) configured to receive the extension (9105) during use. The pump units (9115) shown in figs. 9F1-9F2 covers two sides of the breast unit (9110) during use. This is not essential. The pump units (9115) may cover at least a part of each of the two sides of the breast unit (9110). At least one of the sides, for instance two sides, of the pump units (9115) may comprise a guiding flange to assist in attaching the pump unit (9115) and the breast unit (9110) to each other. As in all embodiments of the invention comprising a flange, such a guiding flange may, but need not, comprise an internal volume comprising parts of the breast pump (9000), such as parts of a pump system.

It is clear that breast units like the breast units (9110) shown in figs. 9F1-9F2 may also cooperate with pump units like the pump units (9115) shown in figs. 9E1-9E2 that cover at least a part of each of three sides of breast units. For instance, the pump unit (9115) shown in fig. 9F1 may be adapted to also include at least one and possibly two lateral sections (a first lateral section in the first lateral position and a second lateral section in the second lateral position).

Fig. 9G shows a breast pump (9000) comprising breast unit (9110) and pump unit (9115). The breast unit (9110) comprises an extension (9105) like the extension (7105) discussed in relation to fig. 7. Reference is made here to fig. 7 and the corresponding discussion. However, whereas in fig. 7 the extension (7105) runs in the proximal-distal direction, the extension (9105) in fig. 9G runs in the lateral direction. In fig. 9G, the pump unit (9115) comprises a groove (9130) configured to at least partially surround the extension (9105) during use. The pump unit (9115) shown in fig. 9G covers two sides of the breast unit (9110) during use. This is not essential. The pump unit (9115) may cover at least a part of each of the two sides of the breast unit (9110). At least one of the sides, for instance two sides, of the pump unit (9115) may comprise a guiding flange to assist in attaching the pump unit (9115) and the breast unit (9110) to each other. As in all embodiments of the invention comprising a flange, such a guiding flange may, but need not, comprise an internal volume comprising parts of the breast pump (9000), such as parts of a pump system.

In each of the embodiments in fig. 9 the pump unit (9115) may comprise a use interface. The user interface may be positioned on a surface of the pump unit (9115) that is schematically shown to be facing in the top direction. For instance, a user interface may be positioned in a distal-top position (indicated by `UI' in fig. 9B1) or lateral-top position on a pump unit. This position has the advantage that the distal part may help blocking the view of a user's breast to a person other than the user. In fig. 6 this would correspond to the area distal (relative to a user) from the second visually transparent part (6085). A lateral-top position may be useful, particularly if the user interface is in a second lateral-top position (indicated by `UI' in fig. 9B4) for a breast pump for a lefthand breast or in a first lateral-top position for a breast pump for a righthand breast. In either of these positions the user interface has a reduced risk of being obscured by clothing during use. In fig. 6 this would correspond to the areas to the left and right from the second visually transparent part (6085). In general, positioning involving a top position would allow a user easy access to the interface during use.

In fig. 9 all pump units (9115) are shown as single elements. However, this is not essential. In embodiments, pump units may comprise multiple sections. One, two or more, or all of these sections may removably attachable to a breast unit (9110). For instance, in an embodiment a pump unit comprises a pump section and a user interface section separate from the pump section. The pump section, the user interface or both may be removably attachable to a breast unit. In another embodiment parts of the pump system are distributed over multiple, separate pump sections. For instance, there may be a section comprises batteries and a section comprising a motor. One, two or more, or all of these pump sections may removably attachable to a breast unit (9110). Having one or more separate pumps sections may be combined with a user interface that is either comprised in one of the pump sections or that is separate from each of the pump sections. In general, parts may be distributed over one or more sections and units may comprise one or more sections. The distribution of parts enables providing a user with a view of a nipple during use. The shape of a part comprising a milk chamber may adapted to provide space for this distribution of these parts, sections, and units.

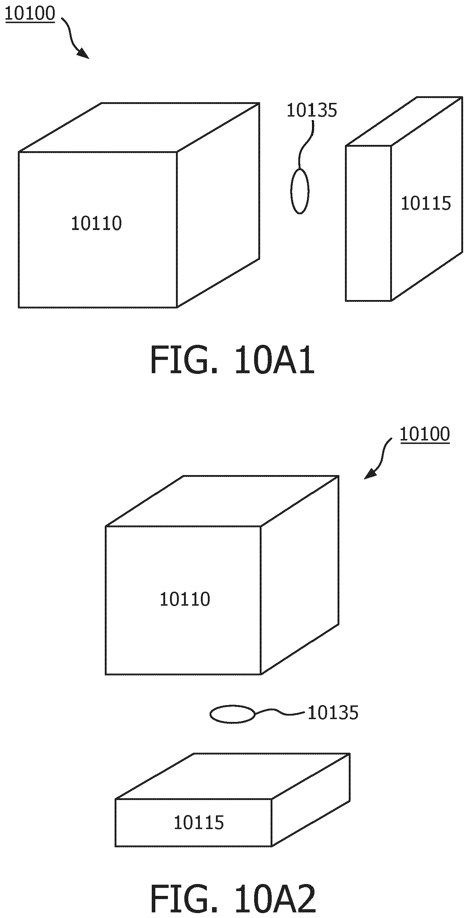

Figs. 10A1-10B2 schematically show different relationships between breast units and pump units in breast pumps according to the invention, wherein the breast pumps comprise membranes that are positioned to not interfere with a users' view of nipples during use. Figs. 10A1-10B2 show breast pumps (10100) comprising breast units (10110) and pump units (10115). Also shown are membranes (10135). The membranes (10135) are configured to apply a pressure profile provided by the pump units (10115) to nipple chambers (not shown) in the breast units (10110).

Figs. 10A1-10A2 are similar to figs. 9B1-9B2. Figs. In fig. 10A1 membrane (10135) is shown in a distal position. In fig. 10A2 membrane (10135) is shown in a bottom position. In either of these positions the membrane (10135) it does not interfere with a user's view of a nipple in a nipple chamber during use.

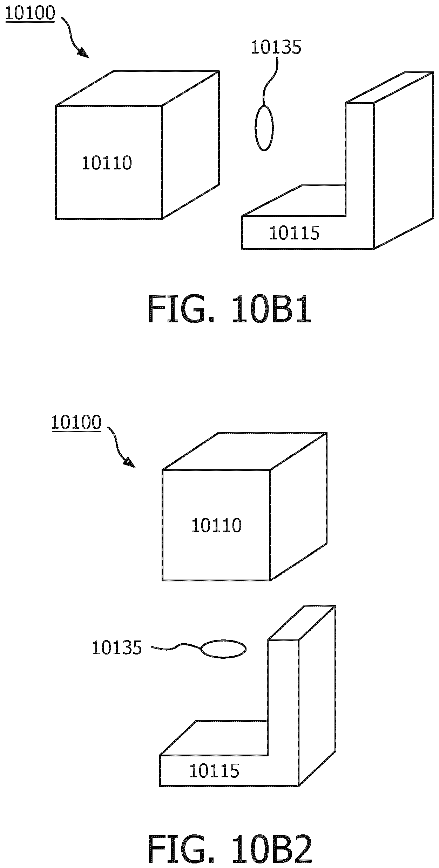

Figs. 10B1-10B2 are similar to fig. 9C1. In fig. 10B1 membrane (10135) is shown in a distal position. In fig. 10B2 membrane (10135) is shown in a bottom position. In either of these positions the membrane (10135) it does not interfere with a user's view of a nipple in a nipple chamber during use.

In figs. 10A1-10A2 the pump units (10115) cover one side of the breast units (10110). As explained in relation to fig. 9 this is not essential. In figs. 10B1-10B2 the pump units (10115) cover two sides of the breast units (10110). As explained in relation to fig. 9 this is not essential.

Based on the above (including fig. 9 and its description) it is clear that there are additional positions in which a membrane (10135) does not interfere with a user's view of a nipple during use. A membrane may, for instance, be positioned in each of the first lateral position and the second lateral position. A membrane may also be a combinations of positions, such as distal-bottom (see also options (a)-(d) listed above). A membrane may also cooperate with a pump unit covering at least a part of two sides or three sides of a breast unit and possibly having a more complex configuration, for instance a configuration as or similar to the configuration of the pump unit (9115) in fig. 9F1. Of course, the position of a membrane should match the position of a pump unit. After all, a membrane cannot be actuated without a pump unit unless the two are functionally coupled during use.

## Claims

1. A breast pump for expressing milk from a nipple of a breast, the breast pump comprising a pump system to provide a pressure profile to be applied to at least a part of the breast and wherein the breast pump is configured to be received in a user's bra during use,
**characterized in that**,
the breast pump is configured such that, during use, no part of the breast pump blocks a user's unobstructed view of the nipple.

2. The breast pump as claimed in claim 1, wherein the breast pump is configured such that, during use,
(i) the breast pump comprises a nipple chamber for receiving the nipple,
(ii) the nipple chamber comprises a first visually transparent part, wherein the first visually transparent part is positioned to allow the user the unobstructed view of a nipple received in the nipple chamber.

3. The breast pump as claimed in claim 2, wherein the breast pump comprises an outer wall portion, not part of the nipple chamber and defining an outer boundary of the breast pump, and wherein the outer wall portion comprises a second visually transparent part, wherein the breast pump is configured such that, during use, the first visually transparent part and the second visually transparent part are aligned to allow the user the unobstructed view of the nipple.

4. The breast pump as claimed in any one of claims 1-3, wherein, during use, the pump system is positioned to not interfere with the unobstructed view.

5. The breast pump as claimed in any one of claims 1-4, wherein the breast pump comprises a milk chamber for receiving expressed milk and wherein the milk chamber is configured to at least partially extend lower than a volume for receiving the nipple during use.

6. The breast pump as claimed in claim 5, wherein the milk chamber comprises a protruding part configured to extend below a volume for receiving the nipple during use and wherein, during use, at least a part of the pump system at least partially surrounds the protrusion.

7. The breast pump as claimed in any one of claims 1-6, wherein the breast pump comprises a breast unit configured to receive the at least a part of the breast and a pump unit comprising the pump system, wherein the pump unit is not comprised in the breast unit.

8. The breast pump as claimed in claim 7, wherein the breast unit and the pump unit are removably attachable to each other.

9. The breast pump as claimed in any one of claims 7-8, wherein the breast unit comprises the milk chamber.

10. The breast pump as claimed in claim 9, wherein the milk chamber is defined by a breast shield and a milk container, wherein the breast shield and the milk container are removably attachable to each other.

11. The breast pump as claimed in any one of claims 1-10, wherein the breast pump comprises a membrane configured to communicate the pressure profile to the at least part of the breast during use and wherein, during use, the membrane is positioned to not interfere with the unobstructed view.

12. The breast pump as claimed in any one of claims 1-11, wherein the breast pump comprises a user interface configured to allow the user to:
(i) control an operation of the breast pump;
(ii) receive information from the breast pump, or
control an operation of the breast pump and receive information from the breast pump,
wherein, during use, the user interface is positioned to not interfere with the unobstructed view.

13. The breast pump as claimed in 12, wherein the pump unit comprises the user interface.

14. A milk container as defined in claim 10.

15. A pump unit as defined in any one of claims 7-8.
